# EUROPEAN PATENT SPECIFICATION

(11) **EP 1 906 767 B1**
(45) Date of publication and mention of the grant of the patent: **04.04.2018**
(21) Application number: 06764972.3
(22) Date of filing: 14.07.2006
(51) Int. Cl.: A61P 9/10, A23L 33/10, A23L 33/19

(54) **LYCOPENE FORMULATIONS FOR THE TREATMENT OF ATHEROSCLEROTIC CONDITIONS**
LYCOPENFORMULIERUNGEN FÜR DIE BEHANDLUNG VON ARTERIOSKLEROSEZUSTÄNDEN
FORMULATIONS DE LYCOPENE POUR LE TRAITEMENT DE MALADIES ATHEROSCLEREUSES

(30) Priority: 21.07.2005 GB 0515035
(43) Date of publication of application: 09.04.2008
(73) Proprietor: CamNutra Limited, Kings Norton Birmingham West Midlands B30 3JN (GB)
(72) Inventor: PETYAEV, Ivan, Cambridge Theranostics Limited, Cambridge CB1 7UY (GB)
(74) Representative: Sutcliffe, Nicholas Robert
(86) International application number: PCT/GB2006/002629
(87) International publication number: WO 2007/010216

(56) References cited:
- EP-A- 0 818 225
- EP-A2- 0 986 963
- WO-A-00/66078
- WO-A-01/26486
- WO-A2-01/89542
- WO-A2-03/019198
- US-A1- 2005 106 219

## Description

### Field of the Invention

This invention relates to the production of compounds useful in the treatment of atherosclerotic and cardiovascular disorders, in particular compounds that inhibit the activity of lipid oxidising abzymes.

### Background to the Invention

Lipid oxidation is one of the main processes leading to the conversion of circulating lipoproteins into highly atherogenic factors [Goto Y. (1982) supra, Halliwell B., and J.M.C. Gutteridge, (1989) supra, Schultz D., and Harrison D.G. (2000) supra]. The principal cause of lipid oxidation is catalytic antibodies or abzymes (see WO03/017992, WO03/019196 and WO03/019198) which are found in atherosclerotic lesions and in the serum of individual with atherosclerotic conditions. These abzymes (which are known as AtheroAbzymes™) are elicited in response to Chlamydia infection, and cross-react with the circulating lipoproteins to produce oxidised atherogenic factors. AtheroAbzymes™ are a key pathogenic factor in the development of atherosclerosis and are an important target for therapeutic intervention for cardiovascular and atherosclerosis-related conditions. The use of the non-hydrophilic anti-oxidant lycopene in the treatment of atherosclerotic and/or cardiovascular disorders has been described (see for example WO 01/26486, WO 01/89542, EP 0 986 963, WO 00/66078 or EP 0 818 225).

### Summary of the Invention

It has now been surprisingly found that compounds, in particular amphipathic or lipophilic anti-oxidant compounds, which display little or no abzyme inhibition activity, can be made into potent abzyme inhibitors by increasing their solubility in aqueous solvents, for example by admixing with a solubilising agent, which is isolated whey protein. An aspect of the invention provides a method of identifying and/or obtaining a composition which inhibits lipid oxidising abzymes comprising:
contacting a lipid oxidising abzyme with a composition comprising a test compound and isolated whey protein, and; determining the inhibition of abzyme activity by said composition, wherein inhibition of abzyme activity is indicative of the composition being useful in the treatment of an atherosclerotic condition. Inhibition of abzyme activity is indicative that the compound may be useful in the treatment of an atherosclerotic condition, such as coronary heart disease (CHD).

The ability of the test compound to inhibit abzyme activity is increased by the presence of the solubilising agent. The test compound may for example show little or no inhibition of abzyme activity in the absence of the solubilising agent.

Suitable test compounds include non-hydrophilic anti-oxidants, for example carotenoids such as lycopene. Suitable solubilising agents include soluble proteins, for example lactoproteins. The solubilising agent according to the invention is isolated whey protein.

The invention further provides a composition comprising isolated whey protein and lycopene for use in the treatment of an atherosclerotic condition, wherein the composition is obtainable by a process comprising dissolving lycopene in a solvent and mixing the solution with an aqueous solution of isolated whey protein.

The invention also provides a use of a composition comprising isolated whey protein and lycopene in the manufacture of a composition for the treatment of an atherosclerotic condition, wherein the composition is obtainable by a process comprising dissolving the lycopene in a solvent and mixing the solution with an aqueous solution of isolated whey protein.

The following is also disclosed:
A method of increasing the ability of a test compound to inhibit lipid oxidising abzymes comprising:
   admixing the test compound with the solubilising agent. A method of producing a composition for use in treatment of an atherosclerotic condition comprising:
      admixing an non-hydrophilic anti-oxidant, for example a carotenoid such as lycopene, with the solubilising agent. A method of treatment of an atherosclerotic condition comprising;
      administering to an individual a composition comprising the solubilising agent and a non-hydrophilic anti-oxidant. A method of making a composition for use in the treatment of an atherosclerotic condition comprising;
      admixing the solubilising agent and a non-hydrophilic anti-oxidant.

Therapies involving administering compositions as described herein may have the effect of reducing abzyme mediated lipid oxidation activity in the vascular system of an individual and thereby ameliorating or alleviating the symptoms of the atherosclerotic condition.

In some embodiments, a method described herein may additionally comprise the step of determining the abzyme mediated lipid oxidation activity of a sample obtained from the individual before, during and/or after said treatment.

### Description of the Tables

Table 1 shows the effect of different formulation of lycopene on its abzyme inhibition activity.

Table 2 shows a Rose-Blackburn Questionnaire.

Table 3 shows the effect of lactolycopene on levels of abzyme mediated lipid oxidation activity and disease severity in patients with coronary heart disease (CHD).

Table 4 shows the comparative effect of a different lycopene preparation, Lyc-O-Mato™, on levels of abzyme mediated lipid oxidation activity and disease severity in patients with CHD.

Table 5 shows comparative effect on the activity of lipid oxidising abzymes of bread and scone food matrixes incorporating either lactolycopene (INNEOV) or Lyc-O-Mato™.

### Detailed Description of the Invention

Compounds which are useful in combination with the solubilising agent in the treatment of atherosclerotic conditions may be identified by contacting a lipid oxidising abzyme with a composition comprising a test compound and the solubilising agent, and; determining the inhibition of abzyme activity by the composition.

A lipid oxidising abzyme (or AtheroAbzyme™) is a catalytic antibody, in particular an IgG molecule, which binds to lipids and catalyses the oxidation thereof. Lipid oxidising abzymes may be produced in an individual in response to Chlamydia infection, and may bind to or be reactive with Chlamydia cells i.e. abzymes may catalyse the oxidation of Chlamydia cells.

Lipid oxidising abzymes bind and oxidise Chlamydia cells, such as human *Chlamydia pneumoniae* cells or ovine Chlamydia cells from species belonging to the *Chlamydia psittaci* group, which includes *Chlamydia psittaci* and *Chlamydia pneumoniae.* Abzymes which bind and oxidise Chlamydia cells may cross-react with host lipoproteins, and are therefore potent atherogenic agents.

Suitable lipid oxidising abzymes may be obtained from atherosclerotic lesions or from the serum of an individual having an atherosclerotic condition. Alternatively, lipid-oxidising abzymes may be obtained using conventional immunological techniques. In some embodiments, the abzymes may be in an isolated or partially isolated form. Suitable methods for isolating and/or obtaining lipid oxidising anti-Chlamydia abzymes suitable for use in the present methods are well-known in the art and are described in WO03/017992, WO03/019196 and WO03/019198.

The measurement of activity of lipid oxidising abzymes is described in more detail below.

Abzyme activity in the presence of the composition comprising the test compound and the solubilising agent may be compared with abzyme activity in comparable reaction medium and conditions in the absence of the composition and, optionally, in the presence of test compound alone. A decrease in activity in the presence of the composition relative to the absence of the composition and/or the presence of the test compound alone, is indicative that the test compound is an inhibitor of abzyme activity in the presence of the solubilising agent. The solubilising agent suitable for use as described herein is a hydrophilic compound that is soluble in aqueous solution. The solubilising agent may be insoluble in organic solvents.

Suitable hydrophilic solubilising agents not according to the inventions include soluble proteins, in particular lactoproteins, such as casein, betalactoglobulin, alpha-lactalbumin, and serum albumin. Whey protein is used as the solubilising agent.

Whey protein is a collection of globular proteins which are naturally found in milk. It is isolated from whey, which is a by-product of cheese manufacture. It is a mixture of betalactoglobulin (∼65%), alpha-lactalbumin (∼25%), and serum albumin (∼8%), which are soluble in their native forms, independent of pH. Whey protein is commercially available from a number of suppliers (e.g. Eurosérum, France).

A suitable test compound may be any non-hydrophilic (i.e. amphipathic or lipophilic) small chemical entity or other molecule. Suitable test compounds may be selected from compound collections and designed compounds, for example using combinatorial chemistry.

Suitable test compounds include amphipathic or amphiphilic compounds which comprise a hydrophilic moiety and a hydrophobic or lipophilic moiety and form a self-assembling micelle structure in aqueous solution. Within the micelle structure, the hydrophobic moieties are located in the interior and the hydrophilic or polar moieties face outwards to interact with the polar water molecules.

Particularly suitable test compounds include anti-oxidants. An anti-oxidant inhibits oxidation reactions in an organism, for example by scavenging and detoxifying oxidative species such as free radicals, and thereby limiting the cellular damage caused by these species.

In some embodiments, a method may comprise determining the anti-oxidant activity of a test compound and identifying the compound as an antioxidant. Anti-oxidant activity may be determined in any suitable biochemical or biophysical test system.

Suitable test compounds may include non-hydrophilic anti-oxidants such as lycopene and analogues, salts and derivatives thereof, carotenoids other than lycopene, polyphenols, flavonoids, isoflavones, curcuminoids, ceramides, proanthocyanidins, terpenoids, sterols, phytosterols, sterol esters, tocotrienols, squalenes, and retinoids, or salts, analogues or derivatives thereof which possess anti-oxidant activity.

A salt of a compound may be an acid addition salt in which the base retains the biological effectiveness and properties of the compound and which is physiologically acceptable. Such salts include those formed with inorganic acids such as hydrochloric acid, hydrobromic acid, sulfuric acid, nitric acid, phosphoric acid and the like, and organic acids such as acetic acid, propionic acid, glycolic acid, pyruvic acid, oxalic acid, malic acid, malonic acid, succinic acid, maleic acid, fumaric acid, tartaric acid, citric acid, benzoic acid, cinnamic acid, mandelic acid, methanesulfonic acid, ethanesulfonic acid, p-toluenesulfonic acid, salicylic acid and the like.

In some preferred embodiments, the test compound is a lycopene compound, for example lycopene or a derivative of lycopene which possesses similar biological properties to lycopene. Lycopene is an open-chain unsaturated C₄₀ carotenoid of structure I (Chemical Abstracts Service Registry Number 502-65-8) which occurs naturally in plants such as tomatoes, guava, rosehip, watermelon and pink grapefruit.

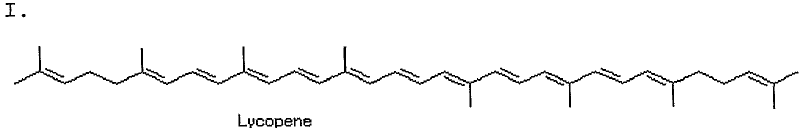

Lycopene for use as described herein may comprise one or more different isomers. For example, lycopene may comprise at least 10%, at least 20%, at least 30%, at least 40%, at least 50%, at least 60%, at least 70% , at least 80% , at least 90%, or at least 95% (Z)-isomers, (all-E)-isomers, or cis-isomers, such as *5-cis-* or *9-cis-* or 13*-cis-isomers*, which have improved bioavailability relative to trans isomers. *Trans* isomers may isomerise into cis forms in *vivo*, or during storage and processing.

Derivatives of lycopene which possess similar biological properties to lycopene may include, for example, carotenoids such as retinoic acid, synthetic acyclo-retinoic acid; or 1-HO-3', 4'-didehydrolycopene, 3, 1'-(HO)2-gamma-carotene, 1,1'-(HO)2-3, 4, 3', 4'-tetradehydrolycopene, and 1, 1'-(HO)2-3, 4-didehydrolycopene.

A lycopene compound for use as described herein may be natural i.e. obtained from a natural source, for example, extracted from a plant such as tomato or melon. A range of methods for extracting, concentrating and/or purifying lycopene compounds from plants are known in the art. For example, solvent extraction using ethanol, DMSO, ethyl acetate, hexane, acetone, soya or other vegetable oil, or non-vegetable oils may be employed.

Lycopene compounds for use as described herein may be synthetic i.e. produced by artificial means, for example, by chemical synthesis. A range of methods for chemical synthesis of lycopene and other carotenoids are known in the art. For example, a three-stage chemical synthesis based on the standard Wittig olefination reaction scheme for carotenoid synthesis may be employed, in which an organic solution of C₁₅ phosphonium methanesulfonate in dichloromethane (DCM) and an organic solution of C₁₀ dialdehyde in toluene are produced, and the two organic solutions are gradually combined with sodium methoxide solution and undergo a condensation reaction to form crude lycopene. The crude lycopene may then be purified using routine techniques, for example by adding glacial acetic acid and deionized water to the mixture, stirring vigorously, allowing the aqueous and organic phases to separate, and extracting the organic phase containing DCM and crude lycopene with water. Methanol is added to the organic phase and the DCM removed via distillation under reduced pressure. The crude methanolic lycopene solution is then be heated and cooled to crystalline slurry that is filtered and washed with methanol. The lycopene crystals may then be recrystalized and dried under heated nitrogen. Synthetic lycopene is also available from commercial suppliers (e.g. BASF Corp, NJ USA).

Synthetic lycopene may comprise an increased proportion of cis isomers relative to natural lycopene. For example, synthetic lycopene may be up to 25% 5-cis, 1% 9-cis, 1% 13-cis, and 3% other cis isomers, whilst lycopene produced by tomatoes may be 3-5% 5-cis, 0-1% 9-cis, 1% 13-cis, and <1% other cis isomers. Since cis-lycopene has increased bioavailability relative to trans-lycopene, synthetic lycopene is preferred in some embodiments.

Derivatives of lycopene as described above may be produced by chemical synthesis analogous to the synthesis described above or by chemical modification of natural lycopene extracted from plant material. The non-hydrophilic anti-oxidant lycopene is formulated with whey protein. Lycopene formulations with whey protein (termed 'lactolycopene') are known in the art (see for example, Richelle et al J. Nutr. 132:404-408, 2002, US 2005/106219 A1 and PCT/EP01/06145) and are commercially available (INNEOV, L'Oréal (UK) Ltd, London). Lactolycopene may be obtained or obtainable by the methods described in these documents or a method described herein.

In some preferred embodiments, a test compound may be identified as having no effect or substantially no effect on abzyme activity in the absence of solubilising agents.

The amount of test compound which may be incorporated into a composition for use in a method of the invention will normally be determined by trial and error depending upon the type of compound used. Typically, from about 0.01 to 100 nM concentrations of putative inhibitor compound may be used, for example from 0.1 to 10 nM.

The composition may comprise 0.05 to 50% by weight of the test compound and 5 to 90% by weight of the solubilising agent.
The solubilising agent and test compound may be present in a weight ratio of from 1:1 to 1000:1, preferably from 2:1 to 250:1 and more preferably about 3:1 to 20:1.

Test compounds which may be used may be natural or synthetic chemical compounds used in drug screening programmes.
Extracts of plants which contain several characterised or uncharacterised components may be used.

The solubilising agent and the anti-oxidant may be admixed together into a composition or may be directly or indirectly linked within the composition (e.g. as a conjugate). The solubilising agent and the anti-oxidant may, for example, be linked by a covalent linkage e.g. via one or more covalent bonds using standard chemical techniques, or a non-covalent linkage, e.g. via antigen/antibody binding.

For example, a composition may be prepared by dissolving the test compound in a solvent and mixing the solution with an aqueous solution of isolated whey protein. The test compound may be dissolved in any pharmaceutically compatible solvent. The solvent is preferably acetone, ethanol or isopropanol. When an aqueous protein solution is mixed with the solvent, a solvent/water ratio by volume of the order of 60/40 is chosen. After mixing, the mixture may be left to stand for 30 to 60 min at a temperature slightly higher than ambient temperature. The solvent may then evaporated to produce a composition in emulsion or dispersion form. Evaporation may be conveniently achieved using reduced pressure (e.g. 200 to 300 mbar). The composition may then be further treated, for example by drying to produce a powder or by heat-treating to produce a gel.

The activity of a lipid oxidising abzyme, including lipid peroxidation activity, may be determined by determining the oxidation of lipid, which may be lipid from a foreign antigen such as a Chlamydia cell, or lipid from another source, which may for example be added as part of an assay method. The accumulation of oxidation products or by-products, such as co-oxidised coupled reporter molecules, may be measured or the disappearance or consumption of substrates such as non-modified lipids or co-substrates such as oxygen. Many methods for determining lipid peroxidation are known in the art and are described, for example, CRC Handbook of Methods for Oxygen Radical Research, CRC Press, Boca Raton, Florida (1985), Oxygen Radicals in Biological Systems. Methods in Enzymology, v. 186, Academic Press, London (1990); Oxygen Radicals in Biological Systems. Methods in Enzymology, v. 234, Academic Press, San Diego, New York, Boston, London (1994); and, Free Radicals. A practical approach. IRL Press, Oxford, New York, Tokyo (1996)

In preferred embodiments, abzyme lipid oxidisation activity may be determined by determining the production (i.e. the presence or amount) of lipid oxidation products, include aldehydes such as malondialdehyde (MDA), (lipid) peroxides, diene conjugates or hydrocarbon gases. Lipid oxidation products may be determined by any suitable method. For example, lipid peroxidation products may be determined using HPLC (Brown, R.K., and Kelly, F.J In: Free Radicals. A practical approach. IRL Press, Oxford, New York, Tokyo (1996), 119-131), UV spectroscopy (Kinter, M. Quantitative analysis of 4-hydroxy-2-nonenal. Ibid.133-145), or gas chromatography-mass spectrometry (Morrow, J.D., and Roberts, L.J. F2-Isoprostanes: prostaglandin-like products of lipid peroxidation. Ibid. 147-157). The production of malondialdehyde (MDA), for example, may be determined, following reaction with 2-thiobarbituric acid (conveniently at 1mM), by measuring absorbance at an appropriate wavelength, for example 525 nm. The determination of abzyme activity is described in more detail in WO03/017992, WO03/019196 and WO03/019198.

Abzyme activity may also be determined using immunological techniques, for example as described in GB0503940.9.

Inhibition of abzyme activity may be identified using conditions in which, in the absence of a positively-testing agent, the abzyme oxidises lipid. Such compounds may be used as agents to inhibit the function of lipid oxidising abzymes, for example in the treatment of atherosclerotic conditions.

A composition having ability to inhibit abzyme activity may be assessed further using one or more secondary screens. A secondary screen may involve testing for abzyme activity in the vascular system or for a biological function of an abzyme, for example, in an animal model. Suitable biological functions which may be assessed in a secondary screen include reduction in size or number of atherosclerotic lesions, or a reduction in other symptoms or effects of an atherosclerotic disorder, such as blood pressure.

Methods of the present invention may include identifying a composition comprising a test compound and the solubilising agent as a composition that inhibits abzyme activity.

The identified composition may be synthesised or manufactured. Optionally, a composition identified as abzyme inhibitor as described herein may be modified to optimise activity or provide other beneficial characteristics, such as increased half-life or reduced side effects upon administration to an individual. Techniques and strategies for the modification of lead compounds are well known in the art.

A composition comprising lycopene, and the solubilising agent isolated whey protein, as described in the claims may be used in the treatment of the human or animal body in the treatment of an atherosclerotic condition.

Such a composition as described in the claims is used for the manufacture of a medicament for use in the treatment of an atherosclerotic condition.

A method of treatment of an atherosclerotic condition may comprise administering a composition as described in the claims to an individual in need thereof.

An atherosclerotic condition includes atherosclerosis and/or one or more of its clinical complications or an associated cardiovascular condition, such as hyperlipidaemia; ischaemic (coronary) heart disease; myocardial ischaemia(angina); myocardial infarction; aneurismal disease; atheromatous peripheral vascular disease; aortoiliac disease; chronic and critical lower limb ischaemia; visceral ischaemia; renal artery disease; cerebrovascular disease; stroke; atherosclerotic retinopathy; hypercoagulative disorder; thrombosis and aberrant blood clotting; and hypertension. Such conditions may be medical or veterinary conditions.

Individuals which may be the subject of methods of the present invention include humans and non-human animals, including domestic animals such as dogs, cats, horses and parrots, farm animals such as sheep and cattle and rare or exotic animals such as elephants and tigers. References to 'human' herein should be understood to include 'non-human animal' except where the specific context dictates otherwise.

Treatment or therapy refers to any administration of a compound in accordance with the invention which is intended to alleviate the severity of an atherosclerotic disorder in a subject, and includes treatment intended to cure the disease, provide relief from the symptoms of the disease and to prevent or arrest the development or onset of the disease in an individual at risk from developing the disease or an individual having symptoms indicating the development of the disease in that individual.

A composition described herein may be formulated, with a pharmaceutically acceptable excipient as described below. Such a composition may be administered to an individual.

Compositions may be formulated for any suitable route and means of administration. Pharmaceutically acceptable carriers or diluents include those used in formulations suitable for oral, rectal, nasal, topical (including buccal and sublingual), vaginal or parenteral (including subcutaneous, intramuscular, intravenous, intradermal, intrathecal and epidural) administration. The formulations may conveniently be presented in unit dosage form and may be prepared by any of the methods well known in the art of pharmacy. Such methods include the step of bringing into association the active ingredient with the carrier which constitutes one or more accessory ingredients. In general the formulations are prepared by uniformly and intimately bringing into association the active ingredient with liquid carriers or finely divided solid carriers or both, and then, if necessary, shaping the product.

For solid compositions, conventional non-toxic solid carriers include, for example, pharmaceutical grades of mannitol, lactose, cellulose, cellulose derivatives, starch, magnesium stearate, sodium saccharin, talcum, glucose, sucrose, magnesium carbonate, and the like may be used. The active compound as defined above may be formulated as suppositories using, for example, polyalkylene glycols, acetylated triglycerides and the like, as the carrier. Liquid pharmaceutically administrable compositions can, for example, be prepared by dissolving, dispersing, etc, an active compound as defined above and optional pharmaceutical adjuvants in a carrier, such as, for example, water, saline aqueous dextrose, glycerol, ethanol, and the like, to thereby form a solution or suspension. If desired, the pharmaceutical composition to be administered may also contain minor amounts of non-toxic auxiliary substances such as wetting or emulsifying agents, pH buffering agents and the like, for example, sodium acetate, sorbitan monolaurate, triethanolamine sodium acetate, sorbitan monolaurate, triethanolamine oleate, etc. Actual methods of preparing such dosage forms are known, or will be apparent, to those skilled in this art; for example, see "Remington: The Science and Practice of Pharmacy", 20th Edition, 2000, pub. Lippincott, Williams & Wilkins. The composition or formulation to be administered will, in any event, contain a quantity of the active compound(s) in an amount effective to alleviate the symptoms of the subject being treated.
Dosage forms or compositions containing active ingredient in the range of 0.25 to 50% with the balance made up from solubilising agent and non-toxic carrier may be prepared.

For oral administration, a pharmaceutically acceptable non-toxic composition is formed by the incorporation of any of the normally employed excipients, such as, for example, pharmaceutical grades of mannitol, lactose, cellulose, cellulose derivatives, sodium crosscarmellose, starch, magnesium stearate, sodium saccharin, talcum, glucose, sucrose, magnesium, carbonate, and the like. Such compositions take the form of solutions, suspensions, tablets, pills, capsules, powders, sustained release formulations and the like. Such compositions may contain 1%-50% active ingredient, more preferably 2-50%, most preferably 5-8%.

Parenteral administration is generally characterized by injection, either subcutaneously, intramuscularly or intravenously. Injectables can be prepared in conventional forms, either as liquid solutions or suspensions, solid forms suitable for solution or suspension in liquid prior to injection, or as emulsions. Suitable excipients are, for example, water, saline, dextrose, glycerol, ethanol or the like. In addition, if desired, the pharmaceutical compositions to be administered may also contain minor amounts of non-toxic auxiliary substances such as wetting or emulsifying agents, pH buffering agents and the like, such as for example, sodium acetate, sorbitan monolaurate, triethanolamine oleate, triethanolamine sodium acetate, etc.

A more recently devised approach for parenteral administration employs the implantation of a slow-release or sustained-release system, such that a constant level of dosage is maintained. See, e.g., US Patent No. 3,710,795.

The percentage of active compound contained in such parental compositions is highly dependent on the specific nature thereof, as well as the activity of the compound and the needs of the subject. However, percentages of active ingredient of 0.1% to 10% in solution are employable, and will be higher if the composition is a solid that will be subsequently diluted to the above percentages.

The effective amount of the compound or its salt to be administered will ultimately be at the discretion of the physician, taking into account the severity of the disease in a particular subject (e.g. a human patient or animal model) and the overall condition of the subject. Suitable dose ranges will typically be in the range of from 0.01 to 20 mg/kg/day, preferably from 0.1 to 10 mg/kg/day.

Repeat doses may be administered at suitable intervals, for example once or twice daily, twice weekly, weekly or monthly.

A formulation of lycopene, and isolated whey protein according to the claims for use in the treatment of an atherosclerotic condition may be comprised in a food product. For example, it may be comprised in bread, cereal, biscuits, butter, spreads (e.g. margarine), cheese, yogurts, beverages or pet foods, for example canned or dry cat or dog food. Other suitable food products will be apparent to a person skilled in the art.

The formulation may be mixed with the ingredients of the food product prior to cooking (e.g. baking) and/or added to the food product after cooking. The results set out herein show that formulations may be incorporated into food products without loss of food quality and remain active after being cooked into food products.

Preferably, the lycopene compound comprised in the food product is a heterologous lycopene compound. For example, the lycopene compound may be a synthetic lycopene compound, as described above, or a natural lycopene compound that is not naturally present in the food product. For example, a lycopene compound from a fruit or vegetable may be incorporated into bread or cereal.

Thus, also disclosed is a food product comprising a lycopene compound for treating an atherosclerotic condition, wherein the lycopene compound is not naturally present in the food product.

A method of making a food product for treating or delaying or preventing the onset of an atherosclerotic condition is also provided, said method comprising:
(i) providing a food product ingredient,
(ii) mixing a lycopene compound with said ingredient
(iii) formulating said ingredient and said lycopene compound into a food product.

Food product ingredients are the staple foodstuffs, such as flour, meat, eggs, gelatine, milk, salt, preservatives, and water which are used to produce food products. Suitable food product ingredients for use in accordance with the present methods are well-known in the art.

Certain aspects and embodiments of the invention will now be illustrated by way of example and with reference to the tables described below.

### Experimental

### Clinical material

Antibodies were extracted from advanced atherosclerotic lesions of human aorta retrieved from two male patients of 53 and 64 years old, during bypass surgery of an abdominal aortal stenosis at the Centre of Cardio-Vascular Surgery of the Medical University of Rostov-na-Donu, Russian Federation. After recovery these samples were immediately put in 30% w/v solution of NaCl and stored at 0-4°C for 1 month prior to examination. In the control experiments it was shown that during this period, the activities of such enzymes as trypsin, catalase, superoxide dismutase, glutathione peroxidase, creatine kinase and lactate dehydrogenase, together with a level of immunoglobulin (IgG) fragmentation and the degree of lipid peroxidation did not significantly change.

The pieces of aorta (approximately 200-400 mg wet weight) were cut into pieces of approximately 10mg each, placed in 5.0ml of PBS with 1% non-ionic detergent Igepal CA-630 and homogenised by a mechanical homogeniser (Ultra-Turrax) at full-power with a 15mm probe three times for 3 seconds each with 20 second cooling intervals. After homogenisation the insoluble components were separated by centrifugation at 5000g for 10 minutes and supernatants were used for analysis.

### Extraction of abzyme enriched atheroma IgG

The antibodies were extracted and analysed separately from the lesions of the two pieces of the abdominal aorta obtained from the four different patients.

The first step was the treatment of the supernatant with protein A attached to cross-linked 4% beaded agarose at 37°C for 30 minutes. After that the immunoglobulin fraction attached to the beads was spun down at 5000g for 10 minutes. The supernatant was decanted. In order to remove any lipoproteins attached to the sedimented immunoglobulins, the samples were re-suspended with 10% of Igepal CA-630. They were then centrifuged at 5000g for 10 minutes and the supernatant was decanted. To remove the detergent three subsequent washings were performed in the excess of the phosphate buffer with centrifugation under the same regime. The removal of lipoprotein from the immunoglobulin fraction was confirmed by the absence of cholesterol in this fraction.

### Testing of Anti-abzyme activity

Samples of the human serum were incubated with different concentrations of lycopene, lycopene/lactoprotein conjugate or alone (control samples) with the presence of atheroma extracted IgG for 16 hours at 37°C at pH 5.6. The level of lipid peroxidation was assessed as a level of MDA concentration which was measured by spectrophotometric method [Draper, H.H. et al Free Radic. Biol. Med. (1993) 15, 353]. This method is based on the formation of a coloured product when malondialdehyde reacts with thiobarbituric acid.

Briefly, the level of abzymes in a sample was determined as follows: Samples of sera were diluted 1:1 by 0.05M acetate buffer pH 4.0 to make the final pH of these samples between 5.6-5.8. 990µl of the diluted serum was mixed with 10µl of the commercial live ovine *Chlamydia* vaccine (Intervet). Samples were incubated overnight (12-16 hours) at 37°C. 250µl of 40% trichloroacetic acid and 250µl of 1mM 2-thiobarbituric acid was added to each sample. All samples were placed in a water bath and boiled for 30 minutes. Samples were cooled down and centrifuged at 3,000g for 10 minutes. The supernatants were collected and their absorption measured at λ 525nm to determine the concentration of malondialdehydes (MDA), which are products of lipid peroxidation.

### ELISA based Abzyme Assay

ELISA assays were performed using Medac materials and reagents, which were used in accordance with the manufacturers instructions.

Briefly, serum samples from patients were treated as described above. 50 *µ*l of sample diluent was pipetted into microtitre well A1 as blank, and 50 *µ*l of the negative control, Positive Control and the diluted patients' samples were pipetted into other microtitre wells. The microplate wells were incubated for 60 min (± 5 min) at 37oC (± 1°C) in a humid chamber and then washed three times with 200*µ*l wash buffer per well. 50 *µ*l of Conjugate was then added to each well and the microplate wells incubated again for 60 min (± 5 min) at 37oC (± 1°C) in a humid chamber and then washed. 50 *µ*l of TMB-Substrate, was added to each well and the microplate wells incubated for 30 min (± 2 min) at 37oC (± 1°C) in a humid chamber. The reaction was stopped by adding 100 *µ*l of Stop Solution, to each well.

Photometric reading was performed at 450 nm (ref. 620 - 650 nm) within 15 min after adding the Stop Solution.

To calculate the results, the OD value of the blank (well A1) was subtracted from all other OD values. Preferably, the OD value of the blank was < 0.150, the mean OD value of the Negative Control was < 0.100 and the OD value of the Positive Control was > 0.800. Cut-off = mean OD value of the Negative Control + 0.380. Grey zone = Cut-off ± 10%

### Lycopene Preparations

The preparation of the lycopene-lactoprotein composition was as described in US20020107292. Briefly, 13.3 kg of whey protein isolate were dissolved in 330 1 of demineralized water and the mixture is stirred for 6 hours at 25-30°C. Separately, 550 g of Lycored™ oleoresin (LycoRed Corp NJ USA) comprising 6% of lycopene, was mixed in 438 1 of acetone and the mixture was stirred.

The two solutions were subsequently mixed for 60 min at 30°C. The final mixture was moderately heated and the acetone was driven off at a moderate pressure. Finally, water was partially driven off at a pressure of 40-50 mbar. An aqueous solution of 200 kg of whey protein isolate and of oleoresin was obtained, which was subsequently spray dried.

### Rose-Blackburn questionnaire

The Rose-Blackburn questionnaire is a tool for evaluating the severity of angina. A series of seven important subjective and objective criteria that correlate with the severity of disease are assessed and converted to numerical scores.

Each criterion is given a score from 1-4, if that patient has the relevant symptom or 0, if they do not.

The sum of the scores for all seven symptoms provides the patient's Rose-Blackburn score. The maximum score is 28.

Two of the patients treated in this study had severe angina and scored an average of 3 for each symptom on the questionnaire; the others were less severe and averaged 2 or below.

### Results

### In vitro assays

Lycopene preparations were assayed for abzyme inhibition activity as described above and the results are set out in Table 1.

Lycopene (L9879: Sigma) was found to be ineffective as an abzyme inhibitor in both ELISA and lipid oxidation assays. The composition comprising both lycopene and lactoprotein, (lactolycopene preparation: INNEOV), was found to be a highly potent inhibitor of the abzyme activity.

The lycopene extract Lyc-O-Mato™ (LycoRed Corp NJ USA) was also found to inhibit abzyme activity, but its ID₅₀ was more than 3.5 times higher than for lactolycopene.

### Clinical trial

The effects of two lycopene preparations, Lactolycopene and Lyc-O-Mato™, were assessed in a pilot clinical trial in the treatment of Coronary Heart Disease (CHD).

Altogether seventeen AtheroAbzyme™ positive patients with CHD, 45 - 60 years old, were recruited for this trial. In the first group, comprising of 7 female and 7 male patients, 2-3 dragees of lactolycopene were administered daily with food, which provided 4-6 mg of lycopene.

In the second group, comprising 1 female and 2 male patients, 1 capsule of Lyc-O-Mato™ was administered daily with food which provided 15 mg of lycopene. The treatment in each group lasted for 2 months.

Prior to initiation of therapy, the patients were asked to complete the Rose-Blackburn questionnaire (table 2) to provide a measure of the severity of the disease. The questionnaire was repeated after two months and AtheroAbzyme™ activity was also measured.

After 2 months of treatment with lactolycopene/INNEOV, AtheroAbzyme™ activity was reduced to undetectable levels in all fourteen patients (table 3). Thirteen out of fourteen of the patients exhibited clinical improvement, measured by reductions in 25% in scores on the Rose-Blackburn questionnaire.

In the second group of patients, two months of administration of Lyc-O-Mato™ resulted in a reduced but still detectable level of AtheroAbzyme™ (table 4). The clinical condition of the patients was also slightly improved but to a lesser degree than in the first group: 9% in scores on the Rose-Blackburn questionnaire.

The results of this pilot Clinical Trial demonstrated the effectiveness of lactolycopene preparations not only in inhibiting abzymes in vivo but also in improving the condition of CHD patients.

### Incorporation of Lycopene into a Food matrix

### Incorporation into Bread

The recipe for one loaf of bread was:
yeast ¾ tsp, strong white flour - 400g, sugar - 1 tsp, butter - 15g, milk powder - 1 tsp, salt - 1 tsp, water 280 ml, in which Lactolycopene (LL) (INNEOV) dragees, or Lyc-O-Mato™ capsules (LM) were dissolved.

Seven loaves were baked: one (the control) did not contain lycopene preparations, the second contained 5 dragees of LL, the second contained 5 dragees of LL, the third - 10 dragees of LL and the fourth - 20 dragees of LL, the fifth, sixth and seventh contained amounts of lycopene from Lyc-O-Mato™, LM, capsules equivalent to those of the loaves with LL.

### Testing

Five volunteers who tested blind-folded these bread found no difference in texture or flavour between all four of these loaves. Furthermore, the ability to inhibit the activity of lipid-oxidising abzymes of the bread was tested. This was done using an AtheroAbzyme™ ELISA kit (CTL, UK), following the protocol set out in the manufacturers instructions. Bread comprising Lactolycopene had the significant anti-abzyme activity, whereas the bread comprising Lyc-O-Mato™, LM, or control bread did not (table 5).

### Incorporation into Scones

The recipe for a batch of four scones was:
self-raising flour 175 g, baking powder - 1 tsp, pinch of salt, caster sugar - 20 g, unsalted butter - 37 g, milk - 90 ml in which LL in the form of INEOV dragees was dissolved. Seven batches of scones were baked: one (the control) did not contain lycopene preparations, the second contained 0.5 dragee of LL per scone, the third - 1 dragee of LL per scone and the fourth - 2 dragees of LL per scone, , the fifth, sixth and seventh contained amounts of lycopene from Lyc-O-Mato™ capsules equivalent to those of scones with LL.

### Testing

Five volunteers who tested blind-folded these scones found no difference in texture or flavour between scones from all these four batches. Furthermore, the ability to inhibit the activity of lipid-oxidising abzymes of the scones was tested. This was done using an AtheroAbzyme™ ELISA kit (CTL, UK), following the protocol set out in the manufacturers instructions. Scones comprising Lactolycopene had the significant anti-abzyme activity, whereas the scones comprising Lyc-O-Mato™, LM, or control scones did not (table 5).

**Table 1**

| Lycopene preparations | ID50 | Maximum inhibition in vitro | Maximum inhibition in vivo |
|---|---|---|---|
| PBS (control) | - | 0 | n/a |
| Lycopene | - | 0 | n/a |
| Lyc-O-Mato™ | 3.4µM | 100% | 60% |
| INNEOV | 0.9µM | 100% | 100% |

**Table 2**

| Symptom | Manifestation | | | |
|---|---|---|---|---|
| Duration of chest pain | a few seconds | 3 - 5 min | less than 15 min | less than 30 min |
| Character of chest pain | Sharp | Dull | Spastic | Burning |
| Trigger of chest pain | Significant physical exercise | Ordinary physical exercise | Minimal physical activity | No obvious trigger, pain occurs during rest or sleep |
| Location of chest pain | | | Left half of the chest | Retrosternal area, irradiation to the neck and jaws |
| Response to chest pain attack | Slow down walking | Slow down physical activity | Stop any physical activity | Take nitroglycerine |
| Number of angina attacks per 24 hours | 1 | 2 - 3 | 5 | Up to 10 |
| Nitroglycerine tablets taken per 24 hours | less than 3 | 3 - 5 | 6 - 10 | more than 10 |
| Scale | 1 | 2 | 3 | 4 |

**Table 3**

| Patient | Clinical effect of INNEOV on patients with CHD | | | |
|---|---|---|---|---|
| | before treatment | | after 2 months of treatment | |
| | AtheroAbzyme™ level | Rose-Blackburn score | AtheroAbzyme™ level | Rose-Blackburn score |
| 1 | 30 | 22 | 0 | 16 |
| 2 | 52 | 20 | 0 | 19 |
| 3 | 54 | 14 | 0 | 10 |
| 4 | 75 | 14 | 0 | 14 |
| 5 | 31 | 11 | 0 | 7 |
| 6 | 29 | 13 | 0 | 10 |
| 7 | 26 | 8 | 0 | 5 |
| 8 | 35 | 7 | 0 | 5 |
| 9 | 28 | 13 | 0 | 8 |
| 10 | 22 | 16 | 0 | 9 |
| 11 | 30 | 19 | 0 | 12 |
| 12 | 11 | 13 | 0 | 9 |
| 13 | 24 | 20 | 0 | 15 |
| 14 | 32 | 11 | 0 | 10 |
| | 34 | 14.2 ± 1.18 | 0 | 10.6 ± 1.08 |
| | | | p < 0.001* | p < 0.05* |

| | | | | |
|---|---|---|---|---|
| * statistically significant difference | | | | |

**Table 4**

| Patient | Clinical effect of Lyc-O-Mato™ on patients with CHD | | | |
|---|---|---|---|---|
| | before treatment | | after 2 months of treatment | |
| | AtheroAbzyme™ level | Rose-Blackburn score | AtheroAbzyme™ level | Rose-Blackburn score |
| 1 | 55 | 12 | 17 | 11 |
| 2 | 28 | 6 | 10 | 5 |
| 3 | 14 | 16 | 12 | 15 |
| | 32 + 13.3 | 11.3 + 3.07 | 13 + 2.3 | 10.3 + 3.08 |
| | | | P > 0.05 | P > 0.05 |

**Table 5**

| Food Matrix | Anti-oxidant activity of 1 g of the food matrix containing: | | | | | | | | |
|---|---|---|---|---|---|---|---|---|---|
| | control | 10⁻⁸M of: | | 10⁻⁷M of: | | 10⁻⁶M of: | | 10⁻⁵M of: | |
| | | LL | LM | LL | LM | LL | LM | LL | LM |
| Loaf | 0 | 0 | 0 | 14% | 0 | 15% | 0 | 22% | 0 |
| Scone | 0 | 0 | 0 | 9% | 0 | 29% | 0 | 100% | 0 |

## Claims

1. A composition comprising isolated whey protein and lycopene for use in the treatment of an atherosclerotic condition,
wherein the composition is obtainable by a process comprising dissolving lycopene in a solvent and mixing the solution with an aqueous solution of isolated whey protein.

2. A composition for use according to claim 1 wherein the treatment additionally comprises the step of determining the abzyme mediated lipid oxidation activity of a sample obtained from the individual before, during and/or after said treatment

3. A composition for use according to claim 1 or claim 2 comprised in a food product.

4. A composition for use according to claim 3 wherein the food product is a bread, a cereal, a biscuit, butter, a spread, cheese, yogurt or a beverage.

5. Use of a composition comprising isolated whey protein and lycopene in the manufacture of a composition for the treatment of an atherosclerotic condition,
wherein the composition is obtainable by a process comprising dissolving the lycopene in a solvent and mixing the solution with an aqueous solution of isolated whey protein.

6. Use according to claim 5 wherein the composition is comprised in a food product.

7. Use according to according to claim 6 wherein the food product is a bread, a cereal, a biscuit, butter, a spread, cheese, yogurt, pet food or a beverage.

8. A method of identifying and/or obtaining a composition which inhibits lipid oxidising abzymes comprising:
contacting a lipid oxidising abzyme with a composition comprising a test compound and isolated whey protein, and;
determining the inhibition of abzyme activity by said composition, wherein inhibition of abzyme activity is indicative of the composition being useful in the treatment of an atherosclerotic condition.

9. A method according to claim 8 comprising;
contacting a lipid oxidising abzyme with the test compound, and;
determining the inhibition of abzyme activity by said compound.

10. A method according to claim 9 wherein the test compound does not inhibit abzyme activity in the absence of the isolated whey protein.

11. A method according to any one of claims 8 to 10 comprising formulating the test compound with isolated whey protein to produce said composition.

12. A method according to any one of claims 8 to 11 wherein the test compound is a non-hydrophilic anti-oxidant.

13. A method according to claim 12 wherein the test compound is selected from the group consisting of carotenoids, polyphenols, flavonoids, isoflavones, curcuminoids, ceramides, proanthocyanidins, terpenoids, sterols, phytosterols, sterol esters, tocotrienols, squalenes, and retinoids.

14. A method according to claim 13 wherein the test compound is selected from the group consisting of lycopene, retinoic acid, synthetic acyclo-retinoic acid; or 1-HO-3', 4'-didehydrolycopene, 3, 1'-(HO)2-gamma-carotene, 1,1'-(HO)2-3, 4, 3', 4'-tetradehydrolycopene, and 1, 1'-(HO)2-3, 4-didehydrolycopene.

15. A method according to any one of claims 8 to 14 comprising identifying the composition as an inhibitor of lipid oxidising abzymes.

## Patentansprüche

1. Zusammensetzung, die isoliertes Molkenprotein und Lycopin umfasst, zur Verwendung bei der Behandlung eines Atherosklerose-Leidens,
wobei die Zusammensetzung durch ein Verfahren erhaltlich ist, welches das Lösen von Lycopin in einem Lösungsmittel und das Vermischen der Lösung mit einer wässrigen Lösung von isoliertem Molkenprotein umfasst.

2. Zusammensetzung zur Verwendung nach Anspruch 1, wobei die Behandlung zusätzlich den Schritt des Bestimmens der abzymvermittelten Lipidoxidationsaktivität einer Probe umfasst, die vor, während und/oder nach der Behandlung von dem Individuum erhalten wurde.

3. Zusammensetzung zur Verwendung nach Anspruch 1 oder Anspruch 2, die in einem Nahrungsmittelprodukt umfasst ist.

4. Zusammensetzung zur Verwendung nach Anspruch 3, wobei das Nahrungsmittelprodukt ein Brot, eine Cerealie, ein Keks, Butter, ein Aufstrich, Käse, Joghurt oder ein Getränk ist.

5. Verwendung einer Zusammensetzung, die isoliertes Molkenprotein und Lycopin umfasst, bei der Herstellung einer Zusammensetzung zur Behandlung eines Atherosklerose-Leidens,
wobei die Zusammensetzung durch ein Verfahren erhältlich ist, welches das Lösen des Lycopins in einem Lösungsmittel und das Vermischen der Lösung mit einer wässrigen Lösung von isoliertem Molkenprotein umfasst.

6. Verwendung nach Anspruch 5, wobei die Zusammensetzung in einem Nahrungsmittelprodukt umfasst ist.

7. Verwendung nach Anspruch 6, wobei das Nahrungsmittelprodukt ein Brot, eine Cerealie, ein Keks, Butter, ein Aufstrich, Käse, Joghurt, Tierfutter oder ein Getränk ist.

8. Verfahren zum Identifizieren und/oder Erhalt einer Zusammensetzung, die lipidoxidierende Abzyme hemmt, Folgendes umfassend:
Kontaktieren eines lipidoxidierenden Abzyms mit einer Zusammensetzung, die eine Testverbindung und ein isoliertes Molkenprotein umfasst, und
Bestimmen der Hemmung der Abzymaktivität durch die Zusammensetzung, wobei die Hemmung der Abzymaktivität anzeigt, ob die Zusammensetzung bei der Behandlung eines Atherosklerose-Leidens nützlich ist.

9. Verfahren nach Anspruch 8, Folgendes umfassend:
Kontaktieren eines lipidoxidierenden Abzyms mit der Testverbindung und
Bestimmen der Hemmung der Abzymaktivität durch die Verbindung.

10. Verfahren nach Anspruch 9, wobei die Testverbindung die Abzymaktivität in Abwesenheit des isolierten Molkenproteins nicht hemmt.

11. Verfahren nach einem der Ansprüche 8 bis 10, welches das Formulieren der Testverbindung mit isoliertem Molkenprotein umfasst, um die Zusammensetzung herzustellen.

12. Verfahren nach einem der Ansprüche 8 bis 11, wobei die Testverbindung ein nicht hydrophiles Antioxidans ist.

13. Verfahren nach Anspruch 12, wobei die Testverbindung aus der aus Carotinoiden, Polyphenolen, Flavonoiden, Isoflavonen, Curcuminoiden, Ceramiden, proanthocyanidinen, Terpenoiden, Sterinen, Phytosterinen, Sterinestern, Tocotrienolen, Squalenen und Retinoiden bestehenden Gruppe ausgewählt ist.

14. Verfahren nach Anspruch 13, wobei die Testverbindung aus der aus Lycopin, Retinsäure, synthetischer Acycloretinsäure, oder 1-HO-3', 4'-Didehydrolycopin, 3,1'-(HO)2-γ-Karotin, 1,1'-(HO)2-3,4,3',4'-Tetradehydrolycopin und 1,1'-(HO)2-3,4-Didehydrolycopin bestehenden Gruppe ausgewählt ist.

15. Verfahren nach einem der Ansprüche 8 bis 14, welches das Identifizieren der Zusammensetzung als Hemmer von lipidoxidierenden Abzymen umfasst.

## Revendications

1. Composition comprenant une protéine de lactosérum isolée et du lycopène à utiliser dans le traitement d'un état athérosclérotique,
dans laquelle la composition peut être obtenue par un procédé comprenant la dissolution de lycopène dans un solvant et le mélange de la solution avec une solution aqueuse de protéine de lactosérum isolée.

2. Composition pour une utilisation selon la revendication 1, dans laquelle le traitement comprend en outre l'étape consistant à déterminer l'activité d'oxydation lipidique induite par abzyme d'un échantillon obtenu à partir de l'individu avant, pendant et/ou après ledit traitement.

3. Composition pour une utilisation selon la revendication 1 ou la revendication 2, comprise dans un produit alimentaire.

4. Composition pour une utilisation selon la revendication 3, dans laquelle le produit alimentaire est du pain, une céréale, un biscuit, du beurre, une pâte à tartiner, un fromage, un yaourt ou une boisson.

5. Utilisation d'une composition comprenant une protéine de lactosérum isolée et du lycopène dans la fabrication d'une composition pour le traitement d'un état athérosclérotique,
dans laquelle la composition peut être obtenue par un procédé comprenant la dissolution du lycopène dans un solvant et le mélange de la solution avec une solution aqueuse de protéine de lactosérum isolée.

6. Utilisation selon la revendication 5, dans laquelle la composition est comprise dans un produit alimentaire.

7. Utilisation selon la revendication 6, dans laquelle le produit alimentaire est du pain, une céréale, un biscuit, du beurre, une pâte à tartiner, un fromage, un yaourt, un aliment pour animaux de compagnie ou une boisson.

8. Procédé d'identification et/ou d'obtention d'une composition qui inhibe les abzymes d'oxydation lipidique, comprenant les étapes consistant à :
mettre en contact un abzyme d'oxydation lipidique avec une composition comprenant un composé d'essai et une protéine de lactosérum isolée, et ;
déterminer l'inhibition de l'activité d'abzyme par ladite composition, dans lequel l'inhibition d'activité d'abzyme indique que la composition est utile dans le traitement d'une pathologie athérosclérotique.

9. Procédé selon la revendication 8, comprenant les étapes consistant à ;
mettre en contact un abzyme d'oxydation lipidique avec le composé d'essai, et ;
déterminer l'inhibition d'activité d'abzyme par ledit composé.

10. Procédé selon la revendication 9 dans lequel le composé d'essai n'inhibe pas l'activité d'abzyme en l'absence de la protéine de lactosérum isolée.

11. Procédé selon l'une quelconque des revendications 8 à 10, comprenant la formulation du composé d'essai avec une protéine de lactosérum isolée pour produire ladite composition.

12. Procédé selon l'une quelconque des revendications 8 à 11, dans lequel le composé d'essai est un anti-oxydant non hydrophile.

13. Procédé selon la revendication 12, dans lequel le composé d'essai est choisi dans le groupe constitué de caroténoïdes, polyphénols, flavonoïdes, isoflavones, curcuminoïdes, céramides, proanthocyanidines, terpénoïdes, stérols, phytostérols, esters de stérol, tocotriénols, squalènes, et rétinoïdes.

14. Procédé selon la revendication 13, dans lequel le composé d'essai est choisi dans le groupe constitué de lycopène, d'acide rétinoïque, d'acide acyclo-rétinoïque synthétique ; ou parmi 1-HO-3',4'-didéhydrolycopène, 3,1'-(HO)2-gamma-carotène, 1,1'-(HO)2-3,4,3',4'-tétradéhydrolycopène, et 1,1'-(HO)2-3,4-didéhydrolycopène.

15. Procédé selon l'une quelconque des revendications 8 à 14, comprenant l'identification de la composition en tant qu'inhibiteur d'abzymes d'oxydation lipidique.
